# EUROPEAN PATENT APPLICATION

(11) **EP 2 597 465 A1**
(43) Date of publication of application: **29.05.2013**
(21) Application number: 12173564.1
(22) Date of filing: 26.06.2012
(51) Int. Cl.: G01N 33/68

(54) **TnT and H-FABP for diagnosis of cardiac damage in infection**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 6006 Luzern (CH); Zdunek, Dietmar, 82327 Tutzing (DE)
(74) Representative: Dick, Alexander

(57) **Abstract**

The present invention relates to a method for diagnosing in a subj ect whether cardiac damage is associated with systemic infection. The method is based on the determination of the amount(s) of a cardiac troponin and/or of H-FABP (Heart-type fatty acid binding protein) in a sample from a subject suspected to suffer from systemic infection, and on the comparison of the, thus, determined amount(s) to a reference amount (reference amounts). Further envisaged are kits and devices adapted to carry out the method of the present invention.

## Description

The present invention relates to a method for diagnosing in a subj ect whether cardiac damage is associated with systemic infection. The method is based on the determination of the amount(s) of a cardiac troponin and/or of H-FABP (Heart-type fatty acid binding protein) in a sample from a subject suspected to suffer from systemic infection, and on the comparison of the, thus, determined amount(s) to a reference amount (reference amounts). Further envisaged are kits and devices adapted to carry out the method of the present invention.

Sepsis is the leading caused of death, specifically in critically ill patients, it is caused by the manifestation of the immune und inflammatory response to systemic infection (Hunter J.D et al Brit J Anaesth. 2010: 104: 3 -11).

A key element in SIRS and sepsis is the development of cardiac damage which is completely different from the cardiac damage seen in patients with ischemic cardiomyopathy where systolic dysfunction as indicated by reduced left ventricular ejection fraction (LVEF) represents a key diagnostic information. In contrast, cardiac involvement in systemic infection is characterized by myocardial depression. One component of myocardial depression is a significant increase in end-diastolic and end-systolic volume (e.g. end-diastolic from 120 ml to 180 ml and end-systolic from 50 ml to 120 ml) when normal and diseased hearts are compared (see Hunter J. et al).

The etiology of myocardial depression resulting in myocardial dysfunction is believed to be profound changes in microcirculation resulting in endothelial dysfunction and maldistribution of blood flow (Hinshaw L.B. Crit Care Med 1996: 24:1072 - 8). In contrast, myocardial blood flow in larger arteries has been found to be normal. The mediators causing myocardial depression are multiple and candidates include inflammatory cytokines, endotoxin and nitric oxide. There is also evidence that myocardial dysfunction includes adaptive responses which will allow full recovery (Hunter J.D. et al).

These is little known about cardiac status in individuals with systemic infection beyond the fact that troponin T and NT-pro BNP are markers of poor prognosis.

EP 2 439 535 A2 discloses a method for monitoring the treatment of a cardiac disorder with an anti-inflammatory drug in a diabetes patient having amounts of a natriuretic peptide and GDF-15 thereof that provide contradictory information. The method is based on the determination of the amount of a cardiac troponin or a variant thereof and/or H-FABP in at least two samples from the patient. Based on the comparison of the amounts in the at least two samples, it is possible to assess whether the treatment of the cardiac disorder was successful.

Yan et al. 2009 (European Journal of Pharmacology 616, 244-250) discloses that in patients suffering from pulmonary infection induced multiple organ dysfunction syndrome H-FABP levels are elevated relative to controls, indicating that H-FABP is a useful diagnostic marker for organ dysfunction.

McLean et al. 2008 (Crit Care. 2008; 12{3): 215) reviews the use of cardiac markers in an ICU setting and discloses that H-FABP had a better discriminatory ability for pulmonary embolism-related complications than cTnT and N-terminal-proBNP.

Pulset al. 2007 (European Heart Journal, 28, 224-229) discloses that H-FABP permits early risk stratification of pulmonary embolism.

WO 2009/150181 discloses that human heart-type fatty acid binding protein is a marker for myocardial hibernation, and that cardiac troponins can be used as marker for necrosis. However, there is no indication that the markers can be used as markers in patients with systemic infection and/or sepsis.

As set forth above, systemic infection may cause cardiac damage. Since the cardiac damage caused systemic infection is completely different from the cardiac damage seen in patients without systemic infection, it is important to identify patients suffering from cardiac damage caused by systemic infection, since these patients are in need of a treatment that aims to prevent further damage to the heart.

The technical problem underlying the present invention can be seen as the provision of means and methods for reliably and efficiently diagnosing in a subject whether cardiac damage is caused by systemic infection. The technical problem is solved by the embodiments characterized in the claims and herein below.

Accordingly, the present invention relates to a method for diagnosing in a subject suffering from systemic infection, whether cardiac damage is associated with, preferably caused by systemic infection, comprising the steps of
a) determining the amount(s) of the marker cardiac troponin and/or of the marker H-FABP (Heart-type fatty acid binding protein) in a sample from said subject, and
b) comparing the, thus, determined amount(s) to a reference amount (reference amounts), thereby diagnosing whether cardiac damage is associated with, preferably caused by systemic infection,
wherein (i) the reference amount(s) is (are) derived from a subject suffering from systemic infection and pre-existing heart disease, if the subject to be tested suffers from pre-existing heart disease, and/or wherein (ii) the reference amount(s) is (are) derived from a subject suffering from systemic infection, but not suffering from pre-existing heart disease, if the subject to be tested does not suffer from pre-existing heart disease.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein means assessing whether a subject who suffers from systemic infection suffers from cardiac damage associated with, preferably caused by systemic infection, or not. As will be understood by those skilled in the art, such an assessment is usually not intended to be correct for 100% of the subjects to be diagnosed. The term, however, requires that assessment whether a subject suffers from cardiac damage associated with, preferably caused by systemic infection, or not, is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, N ew York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, preferably mammals, and, more preferably, humans. Preferably, the subject in accordance with the present invention is suspected to suffer from systemic infection. More preferably, the subject suffers from systemic infection.

The term "systemic infection" is well known in the art. An "infection" indicates the presence and/or colonization of an infecting species of pathogen in a host organism. In patients with systemic infection, the pathogen is distributed throughout the body rather than concentrated in one area (which is the case in local infection). Thus, the pathogen is present in the circulating blood. Further, internal organs are affected in systemic infection.

An infection in the sense of the present invention, preferably, is a viral, fungus or bacterial infection, and, more preferably, a bacterial infection. The bacterial infection is, preferably, associated with bacteria selected from E coli, staphylococcus aureus, Klebsiella pneumoniae, Streptococci or Pseudomonas aeroginosa. The infection may as well be an infection by a fungus, e.g., selected from Candida albicans, Candida tropicalis or Aspergillus fumigatus. An infection is diagnosed on the basis of assays and criteria generally known to the physician. Preferably, the infection is diagnosed on the basis of a bacterial culture assay, e.g., a culture medium inoculated with a sample from the patient, or based on molecular diagnostic methods. A fungus infection may, for example, be determined based on the generally known test assays such as Septifast.

It is well known in the art, that systemic infection can progress to SIRS ("Systemic inflammatory response syndrome"). In the context of the present invention, it is particularly contemplated that the subject who suffers from systemic infection has developed SIRS, i.e. also suffers from SIRS. "SIRS" in the context of the present invention is a clinical manifestation secondary to an insult of infectious origin. The term "SIRS" preferably, encompasses SIRS as defined on the ACCP/SCCM Consensus Conference Definitions (1992/2003) (see e.g. American College of Chest Physicians/Society of Critical Care Medicine Consensus Conference: definitions for sepsis and organ failure and guidelines for the use of innovative therapies in sepsis. Crit Care Med. 1992 Jun;20(6):864-74)). Preferably, a patient is suspected to suffer from SIRS, if the patient displays at least 2 symptoms of the following a) to d), preferably of the following a) to e): a) white blood cell count (WBC) > about 12,000/ µ/L or < about 4000/µ/L, b) body temperature > about 38 0C or < about 36 0C, c) heart rate > about 90 beats/minute, d) a respiratory rate > about 20 breaths/minute or a partial pressure of CO2 of less than about 32 mm Hg, and e) more than 10% immature white blood cells among the counted white blood cells. The white blood cell count is usually determined by automated counting devices. As far as children are concerned, the consensus criteria for diagnosing SIRS in a child are disclosed in Goldstein et al. (Goldstein 2005, Pediatr. Crit Care Med 2005, 6(1),2- 8; see in particular Tables 2 and 3).

A subject who suffers from systemic infection and from SIRS suffers from sepsis. In the context of the present invention, it is, thus, preferred that the subject suffers from sepsis. This applies to the subject to be tested and the reference subject. Sepsis may be determined by the aforementioned criteria for SIRS and at least two of the following criteria in addition: a) presence of leucocytes in a physiologically sterile body fluid; b) peritonitis or perforated viscus; c) pneumonia with focal opacification or petechiae, purpura, or purpura fulminans; d) bacteriemia. Further symptoms or particularly severe complications accompanying SIRS or sepsis are described in standard text books of medicine such as Stadmen or Pschyrembl. Sepsis can proceed to severe sepsis, septic shock, refractory septic shock or multi-organ dysfunction syndrome (MODS). The latter conditions are associated with organ dysfunction, e.g. the cardiovascular, the renal, the respiratory, the cerebral or the hematologic system.

Severity of systemic infection can be classified by using the so called APACHE II score. The APACHE II Score includes the assessment of temperature, mean blood pressure, heart rate, respiratory rate, arterial pH, oxygenation, serum sodium, serum potassium, hematocrit and white blood cell count. In addition age, chronic health status and the Glasgow coma Score is considered. Various studies indicate that the APACHE II Score is useful in predicting survival, survival rates have been shown to be higher in postoperative cases at the same APACHE II Score level. The APACHE II scoring system is not restricted to the ICU but can also be performed in the emergency room, this system is however time consuming as it cannot differentiate between different organ failures (Kress J.P., Hall J.B. in Harrison Principles of Internal Medicine p 1673 ff). Preferably, the subject is classified as an APACHE II Score of or larger.

In a preferred embodiment of the method of the present invention, the subject suffers from systemic infection and from pre-existing heart disease. Thus, the subject who suffers from systemic infection shall have suffered from heart disease already before and/or at the onset of systemic infection. In this case, the reference amount for the comparison step shall have been obtained from the sample of a subject who also suffers from systemic infection and from pre-existing heart disease (or from a group of such subj ects).

In another preferred embodiment of the method of the present invention, the subject to be tested suffers from systemic infection, but does not suffer from pre-existing heart disease. A subject who does not suffer from pre-existing heart disease did/does not suffer from heart disease before and/or at the onset of systemic infection, i.e. the subject shall be apparently healthy with respect to heart disease at the onset of symptoms of systemic infection. In this case, the reference amount for the comparison step shall have been obtained from the sample of a subject who suffers from systemic infection but who does not suffer from pre-existing heart disease (or from a group of such subjects).

In a preferred embodiment of the method of the present invention, the pre-existing heart disease is coronary heart disease and/or heart failure. Thus, the subject who suffers from pre-existing heart disease shall have suffered from coronary heart disease and/or heart failure at and/or before the onset of systemic infection, whereas the subject who does not suffer from pre-existing heart disease shall not have suffered from coronary heart disease and heart failure at and/or before the onset of systemic infection.

The term "heart failure" is well known in the art. As used herein, the term, preferably, relates to an impaired systolic and/or diastolic function of the heart being accompanied by overt signs of heart failure. Preferably, heart failure referred to herein is also chronic heart failure.

HF can be classified into various degrees of severity.

According to the NYHA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;el-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages C and D of the ACC/AHA classification referred to above. In these stages, the subject shows typical symptoms of heart failure. Accordingly, a subject who suffers from heart failure, suffers from heart failure stage C or D according to the ACC/AHA classification.

Also preferably, the term "heart failure" refers to heart failure classified as NYHA classes III or IV according to the NYHA classification.

The term "coronary heart disease", abbreviated CHD, also called "coronary artery disease" (CAD) or atherosclerotic heart disease, is known to the person skilled in the art. Particularly, CHD is the result of the accumulation of atheromatous plaques within the walls of the arteries that supply the myocardium (the muscle of the heart). While the symptoms and signs of coronary heart disease are noted in the advanced state of disease, most individuals with coronary heart disease show no evidence of disease for decades as the disease progresses before the first onset of symptoms, often a "sudden" heart attack, finally arise. After decades of progression, some of these atheromatous plaques may rupture and (along with the activation of the blood clotting system) start limiting blood flow to the heart muscle.

Preferably, the coronary heart disease is stable. Thus, the term "stable coronary heart disease", preferably, does not include acute cardiovascular syndromes (ACS). Thus, the subject, preferably, does not suffer from ACS. The term "ACS" as used herein includes STEMI (ST-elevation myocardial infarction); NSTEMI (non ST-elevation myocardial infarction) and unstable angina pectoris.

It is further envisaged that the subj ect does not suffer from chronic renal failure, from myocarditis and/or from pulmonary embolism.

In the context of the present invention, it shall be assessed whether a subject who suffers from systemic infection suffers from cardiac damage associated with said systemic infection. The term "cardiac damage" as used herein refers to any kind of reversible or irreversible damage to the myocardium caused by myocardial ischemia. Ischemia may be a reversible or persisting (i.e. permanent) ischemia. Persisting ischemia is characterized in that the myocardium is inappropriately supplied by blood and, thus, hypoxic, even in a resting subject. The reversible ischemia is characterized in that the inappropriate blood supply of the myocardium.

In a preferred embodiment, the term "cardiac damage" relates to cardiac necrosis and/or cardiac dysfunction.

The term "cardiac necrosis" is well understood by the skilled person. As used herein, the term refers to necrotic cells or tissue of the myocardium. Such necrotic cells or tissue areas can be determined electrocardiographic techniques because the necrotic cells or areas shall influence the electric field produced by a physiologically operating, i.e. beating, heart. Low blood pressure, elevated intraventricular pressure or coronary heart diseases as well as cardiac myopathy are well known disorders which result in a reduced perfusion of the myocardium and, consequently, in cardiac necrosis. It is known in the art that cardiac necrosis is irreversible.

As used herein, the term "cardiac dysfunction" (herein also referred to as "myocardial dysfunction") is known by the person skilled in the art. The term relates to any kind of heart dysfunction, more particularly, the term relates to heart dysfunctions affecting the pumping capability of the heart. In particular, the term "cardiac dysfunction" relates to a condition in which myocardial contractility, metabolism and ventricular function are reduced in order to cope with a reduced oxygen supply. A subject who suffers from cardiac dysfunction, preferably, has viable myocardial tissue with chronic reversible contractile dysfunction, which can be improved upon revascularization.

Cardiac dysfunction can be caused by prolonged myocardial hypoperfusion and that persists at least until blood flow is restored. Thus, the myocardial cells affected by myocardial dysfunction are temporarily asleep, but still viable and normal function can be restored after the cells the oxygen supply has been restored. Accordingly, cardiac dysfunction is reversible, whereas cardiac necrosis is irreversible. Of course, cardiac necrosis and cardiac dysfunction may co-exist.

The cardiac damage as referred to herein shall be associated with systemic infection. The person skilled in the art understands what is meant if cardiac damage is considered to be associated with systemic infection (see Werdan et al. Clin Res Cardiol (2011) 100:661- 668). The term "associated with" as used herein, preferably, refers to a temporal and, more preferably, to a causal relationship between cardiac damage and systemic infection. Thus, in a preferred embodiment, the cardiac damage has been caused by systemic infection.

Preferably, cardiac damage is associated with systemic infection, if a there is a causal relationship between cardiac damage and systemic infection. Indication for such causal connection is e.g. a close time-relationship between cardiac damage and systemic infection. In particular, cardiac damage is considered to be associated with, preferably, caused by systemic infection if it occurs within 8 hours, or within 16 hours after the onset of systemic infection (in particular after the onset of sepsis). Also, cardiac damage may be associated with, preferably, caused by systemic infection if it occurs within 1 day, or within 3 days or more after the onset of systemic infection (in particular after the onset of sepsis).

In a preferred embodiment of the present invention, the amount of a cardiac Troponin is determined and the cardiac damage that is diagnosed is cardiac necrosis.

In another preferred embodiment of the present invention, the amount of H-FABP is determined and the cardiac damage that is diagnosed is cardiac dysfunction.

In an even further preferred embodiment of the present invention, both the amounts of H-FABP and a cardiac troponin are determined, and compared to reference amounts. The combined determination of these markers allow for diagnosing both cardiac dysfunction and cardiac necrosis.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The term "cardiac Troponin" refers to all Troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac Troponin refers to Troponin T and/or Troponin I, and, most preferably, to Troponin T. It is to be understood that isoforms of Troponins may be determined in the method of the present invention together, i.e. simultaneously or sequentially, or individually, i.e. without determining the other isoform at all. Amino acid sequences for human Troponin T and human Troponin I are disclosed in Anderson, loc cit and Ferrieres 1998, Clinical Chemistry, 44: 487-493.

The term "cardiac Troponin" encompasses also variants of the aforementioned specific Troponins, i.e., preferably, of Troponin I, and more preferably, of Troponin T. Such variants have at least the same essential biological and immunological properties as the specific cardiac Troponins. In particular, they share the same essential biological and immunological properties if they are detectable by the same specific assays referred to in this specification, e.g., by ELISA Assays using polyclonal or monoclonal antibodies specifically recognizing the said cardiac Troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least about 50%, at least about 60%, at least about 70%, at least about 80%, at least about 85%, at least about 90%, at least about 92%, at least about 95%, at least about 97%, at least about 98%, or at least about 99% identical with the amino sequence of the specific Troponin. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants may be allelic variants or any other species specific homologs, paralogs, or orthologs. Moreover, the variants referred to herein include fragments of the specific cardiac Troponins or the aforementioned types of variants as long as these fragments have the essential immunological and biological properties as referred to above. Preferably, the cardiac troponin variants have immunological properties (i.e. epitope composition) comparable to those of human troponin T or troponin I. Thus, the variants shall be recognizable by the aforementioned means or ligands used for determination of the concentration of the cardiac troponins. Thus, the variants shall be recognizable by the aforementioned means or ligands used for determination of the concentration of the cardiac troponins. Such fragments may be, e.g., degradation products of the Troponins. Further included are variants which differ due to posttranslational modifications such as phosphorylation or myristylation. Preferably the biological property of troponin I and its variant is the ability to inhibit actomyosin ATPase or to inhibit angiogenesis in vivo and in vitro, which may e.g. be detected based on the assay described by Moses et al. 1999 PNAS USA 96 (6): 2645-2650). Preferably the biological property of troponin T and its variant is the ability to form a complex with troponin C and I, to bind calcium ions or to bind to tropomyosin, preferably if present as a complex of troponin C, I and T or a complex formed by troponin C, troponin I and a variant of troponin T. It is known that low concentrations of circulating cardiac troponin may be detected in subj ects at various conditions, but further studies are required to understand their respective role and rate (Masson et al., Curr Heart Fail Rep (2010) 7:15-21).

The term "H-FABP" as used herein refers to the heart fatty acid binding protein, in particular to the human heart fatty acid binding protein. Preferably, the term also includes variants of the heart type fatty acid binding protein. H-FABP is frequently also referred to heart type fatty acid binding protein. H-FABP is also known as FABP3. The cDNA sequence as well the protein sequence of human H-FABP is well known in the art and was first described by Peeters et al. (Biochem. J. 276 (Pt 1), 203-207 (1991)). Moreover, the sequence of human H-FABP can be found, preferably, in Genebank entry U57623.1 (cDNA sequence) and AAB02555.1 (protein sequence). The major physiological of function of FABP is thought to be the transport of free fatty acids, see e.g. Storch et al., Biochem. Biophys. Acta. 1486 (2000), 28-44.

The term "variant" has been defined herein above. In particular, a variant of H-FABP as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of H-FABP, in particular the entire length.
Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labeled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on ElecsysTM analyzers), CBA (an enzymatic Cobalt Binding Assay, available for example on Roche-HitachiTM analyzers), and latex agglutination assays (available for example on Roche-HitachiTM analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and noncovalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provides the desired assessment in a suitable output format. The said result may, preferably, serve as an aid in diagnosing whether a subject who suffers from systemic infection has cardiac damage associated with, preferably caused by systemic infection.

The reference amount to be applied in the context of the present invention, is preferably, derived from i) a sample from a subject (or from samples from a group of subjects) who suffers from systemic infection and pre-existing heart disease, if the subject to be tested also suffers from systemic infection and pre-existing heart disease, or from ii) a sample from a subject who suffers from systemic infection, but who is not suffering from pre-existing heart disease, if the subject to be tested also suffers from systemic infection, but is not suffering from pre-existing heart disease.

Thus, as set forth above, in case that the subject to be tested shall suffers from pre-existing heart disease, the reference subject shall also suffer from pre-existing heart disease. It is contemplated that the extend of the pre-existing heart disease is the test subject is essentially the same as in the reference subject. The extend of the heart disease can be determined without further ado. Preferably, the test and the reference subject (in particular, the test and reference subject suffering from pre-existing heart disease) have essentially the same NT-proBNP level at the onset of systemic infection. Further, it is envisaged that the (reference) subject (or group thereof) have essentially the same H-FABP level and/or cardiac troponin level at the onset of systemic infection (the level is the amount of the marker(s) in a sample from the subject, in particular in blood, serum or plasma sample). Whether two levels are essentially the same can be assessed by the skilled person without further ado. In particular, levels which differ by less than 5%, or less than 3% are regarded to be essentially the same.

The term "reference amount" as used herein refers to an amount which allows for allocation of a subject into either the group of subjects who suffer from cardiac damage associated with systemic infection or into a group of subjects who do not suffer from cardiac damage associated with systemic infection. Such a reference amount can be a threshold amount which separates these groups from each other. A suitable threshold amount separating the two groups can be calculated without further ado by the statistical tests referred to herein elsewhere based on amounts of a the biomarker(s) from either a subject or group of subjects known to suffer from cardiac damage associated with systemic infection, and/or from a subject or group of subjects known not to suffer from cardiac damage associated with systemic infection. Preferred referenced amounts which can be derived from the aforementioned subjects or group of subjects are indicated elsewhere herein.

Preferably, the reference subject is known not to suffer from cardiac damage associated with systemic infection. In this case, an amount of the marker(s) in the sample from the subj ect to be tested which is (are) essentially identical or which is (are) decreased as compared to the reference amount(s) indicates that the subject to be tested does not suffer from cardiac damage associated with systemic infection.

Preferably, the reference subject is known to suffer from cardiac damage associated with systemic infection. In this case, an amount of the marker(s) in the sample from the subject to be tested which is (are) essentially identical or which is (are) increased as compared to the reference amount(s) indicates that the subject to be tested suffers from cardiac damage as a cause of systemic infection, i.e. suffers from cardiac damage associated with systemic infection.

Preferred reference amounts are as follows:
If the subject to be tested suffers from pre-existing heart disease, the reference amount(s) shall be derived from a subject suffering from systemic infection and pre-existing heart disease (or from a group thereof). In this case preferred reference amounts for a cardiac troponin (in particular, for troponin T) which are derived from a subject or a group of subjects known to suffer from cardiac damage associated with systemic infection, are within a range of 7 to 15 pg/ml, or, more preferably, within a range of 10 to 15 pg/ml. Preferably, the reference amount for the cardiac troponin is 10 pg/ml, more preferably, the reference amount is 15 pg/ml. Preferred reference amounts for H-FABP which are derived from a subject or a group of subjects known to suffer from cardiac damage associated with systemic infection, are within a range of 3000 to 4000 pg/ml, or, more preferably, within a range of 3500 to 4000 pg/ml. Preferably, the reference amount for the H-FABP is 3000 pg/ml, more preferably, the reference amount is 3500 pg/ml. In case the subject suffers from decompensated heart failure, the reference amounts may be even higher. Preferred reference amounts for a cardiac troponin (in particular, for troponin T) which are derived from a subject or a group of subjects known to not suffer from cardiac damage associated with systemic infection, are within a range of 3 to 7 pg/ml, or, more preferably, within a range of 3 to 5 pg/ml. Preferably, the reference amount for the cardiac troponin is 5 pg/ml, more preferably, the reference amount is 3 pg/ml. Preferred reference amounts for H-FABP which are derived from a subject or a group of subjects known not to suffer from cardiac damage associated with systemic infection, are within a range of 2200 to 2500 pg/ml. Preferably, the reference amount for the H-FABP is 2400 pg/ml, more preferably, the reference amount is 2200 pg/ml.

If the subject to be tested does not suffer from pre-existing heart disease, the reference amount(s) shall be derived from a subject suffering from systemic infection, but not suffering from pre-existing heart disease, if the subject to be tested does not suffer from pre-existing heart disease (or from a group thereof). In this case preferred reference amounts for a cardiac troponin (in particular, for troponin T) which are derived from a subject or a group of subjects known to suffer from cardiac damage associated with systemic infection, are within a range of 1.5 to 3 pg/ml, or, more preferably, within a range of 2 to 3 pg/ml. Preferably, the reference amount for the cardiac troponin is 1 pg/ml, more preferably, the reference amount is 2 pg/ml. Preferred reference amounts for H-FABP which are derived from a subject or a group of subjects known to suffer from cardiac damage associated with systemic infection, are within a range of 2000 to 2400 pg/ml, or, more preferably, within a range of 2000 to 2200 pg/ml. Preferably, the reference amount for the H-FABP is 2200 pg/ml, more preferably, the reference amount is 2400 pg/ml. Preferred reference amounts for a cardiac troponin (in particular, for troponin T) which are derived from a subject or a group of subjects known to not suffer from cardiac damage associated with systemic infection, are within a range of 0.02 to 1 pg/ml, or, more preferably, within a range of 0.02 to 0.5 pg/ml. Preferably, the reference amount for the cardiac troponin is 0.5 pg/ml, more preferably, the reference amount is 1 pg/ml. Preferred reference amounts for H-FABP which are derived from a subject or a group of subjects known not to suffer from cardiac damage associated with systemic infection, are within a range of 1200 to 1700 pg/ml, or, more preferably, within a range of 1500 to 1700 pg/ml. Preferably, the reference amount for the H-FABP is 1700 pg/ml, more preferably, the reference amount is 1500 pg/ml.

Moreover, the reference amount may define a threshold amount, whereby an amount larger than the threshold shall be indicative for a subject suffering from cardiac damage associated with systemic infection, an amount lower than the threshold amount shall be an indicator for a subject not suffering from cardiac damage associated with systemic infection.

The reference amount applicable for an individual subject may vary depending on various physiological parameters such as age, gender, or subpopulation, as well as on the means used for the determination of the polypeptide or peptide referred to herein. A suitable reference amount may be determined by the method of the present invention from a reference sample to be analyzed together, i.e., simultaneously or subsequently, with the test sample. A preferred reference amount serving as a threshold may be derived from the upper limit of normal (ULN), i.e., the upper limit of the physiological amount to be found in samples derived from a population. The ULN for a given population of subjects can be determined by various well known techniques.

As set forth above, the reference amount(s) is (are) derived from a subject suffering from systemic infection and pre-existing heart disease, if the subject to be tested suffers from pre-existing heart disease, the reference amount(s) is (are) derived from, if the subject to be tested does not suffer from pre-existing heart disease. Alternatively, it is also contemplated that the reference amount(s) to be used in accordance with the aforementioned method is (are) the amount of the biomarker(s), i.e. of the cardiac Troponin and/or of H-FABP, in a sample from the subject that has been obtained before the onset of symptoms of systemic infection. An amount of H-FABP and/or of cardiac troponin in the test sample which is (are) increased as compared to the reference amount indicates that the subj ect suffers from cardiac damage associated with systemic infection. An amount of H-FABP and/or of cardiac troponin in the test sample which is (are) decreased or essentially the same as compared to the reference amount indicates that the subject does not suffer from cardiac damage associated with systemic infection. Preferably, the sample that has been obtained from the subject before the onset of symptoms of systemic infection, has been obtain not more than three months, more preferably, not more than two months, and most preferably, not more that one month before the onset of symptoms of systemic infection.

In a preferred embodiment, the method of the present invention further comprises the step of recommending a therapy , if the subject to be tested suffers from cardiac damage associated with systemic infection. Preferably, the therapy to be recommended is selected from the group consisting of administration of broad sprectrum antibiotics (in particular if the systemic infection is caused by bacteria), administration of dopamine, and application of fluid.

Advantageously, it has been shown in the context of the present invention that H-FABP and cardiac troponins are reliable markers for diagnosing in a subject who suffers from systemic infection whether said subject suffers from cardiac damage associated with systemic infection, or whether said subject does not suffer from cardiac damage associated with systemic infection. In particular, it has been that the amounts of H-FABP and cardiac troponins are generally increased in patients exhibiting systemic infection. Therefore, a reference amount that has not been obtained from a subject suffering from systemic infection is not applicable for carrying out the method according to the present invention. Further, the cardiac status of the subject to be tested prior to the systemic infection has to be taken into account. If the subject has a pre-existing cardiac disease, the reference amount shall be derived from a subject (or group of subjects) who has also a pre-existing heart disease. Moreover, if the subject has no pre-existing cardiac disease, the reference amount shall be derived from a subject (or group of subjects) who has also no pre-existing heart disease. Accordingly, the reference amount for carrying out the diagnosis shall be derived from a subject suffering from systemic infection who has the same cardiac status prior to the onset of systemic infection as the test subject since the use of other reference amounts would lead to a large number of incorrect test results.

In an aspect of the invention, a method for establishing an aid for diagnosing whether a subject suffering from systemic infection is suffering from cardiac damage associated with systemic infection, or not, is contemplated, said method comprising:
a) determining the amount(s) of H-FABP and/or a cardiac troponin in a sample of said subject, said determining comprises (i) bringing the sample into contact with a detection agent that specifically binds to H-FABP and/or with a detection agent that specifically binds to a cardiac troponin for a time sufficient to allow for the formation of a complex of the said detection agent(s) and H-FABP and/or said cardiac troponin from the sample, (ii) measuring the amount of the formed complex, wherein the said amount of the formed complex is proportional to the amount of the marker (H-FABP and/or the cardiac troponin) present in the sample, and (iii) transforming the amount of the formed complex into an amount of reflecting the amount of the marker present in the sample;
b) comparing said amount(s) to a reference amount as specified herein elsewhere; and
c) establishing an aid for diagnosing whether a subject suffering from systemic infection is suffering from cardiac damage associated with systemic infection, or not based on the result of the comparison made in step b).

A suitable detection agent may be, in an aspect, an antibody which is specifically binds to a marker in a sample of a subject to be investigated by the method of the invention. Another detection agent that can be applied, in an aspect, may be an aptamere which specifically binds to the marker in the sample. In yet an aspect the, sample is removed from the complex formed between the detection agent and the marker prior to the measurement of the amount of formed complex. Accordingly, in an aspect, the detection agent may be immobilized on a solid support. In yet an aspect, the sample can be removed from the formed complex on the solid support by applying a washing solution. The formed complex shall be proportional to the amount of the marker present in the sample. It will be understood that the specificity and/or sensitivity of the detection agent to be applied defines the degree of proportion of marker comprised in the sample which is capable of being specifically bound. Further details on how the determination can be carried out are also found elsewhere herein. The amount of formed complex shall be transformed into an amount of marker reflecting the amount indeed present in the sample. Such an amount, in an aspect, may be essentially the amount present in the sample or may be, in another aspect, an amount which is a certain proportion thereof due to the relationship between the formed complex and the amount present in the original sample.

In yet an aspect of the aforementioned method, step a) may be carried out by an analyzing unit, in an aspect, an analyzing unit as defined elsewhere herein.

In an aspect of the method of the invention, the amount determined in step a) is compared to a reference. In an aspect, the reference is a reference as defined elsewhere herein. In yet another aspect, the reference takes into account the proportional relationship between the measured amount of complex and the amount present in the original sample. Thus, the references applied in an aspect of the method of the invention are artificial references which are adopted to reflect the limitations of the detection agent that has been used. In another aspect, said relationship can be also taken into account when carrying out the comparison, e.g., by including a normalization and/or correction calculation step for the determined amount prior to actually comparing the value of the determined amount and the reference. Again, the normalization and/or correction calculation step for the determined amount adopts the comparison step such that the limitations of the detection agent that has been used are reflected properly. In an aspect, the comparison is carried out automatically, e.g., assisted by a computer system or the like.

The aid for diagnosing is established based on the comparison carried out in step b) by allocating the subject either into a group of subjects suffering from cardiac damage associated with systemic infection with a certain likelihood or a group of subjects not suffering therefrom. As discussed elsewhere herein already, the allocation of the investigated subject must not be correct in 100% of the investigated cases. Moreover, the groups of subjects into which the investigated subject is allocated are artificial groups in that they are established based on statistical considerations, i.e. a certain preselected degree of likelihood based on which the method of the invention shall operate. Thus, the method may establish an aid of diagnosis which may, in an aspect, require further strengthening of the diagnosis by other techniques. In an aspect of the invention, the aid for diagnosing is established automatically, e.g., assisted by a computer system or the like.

In an aspect of the method of the invention, said method further comprises a step of recommending and/or managing the subject according to the result of the aid of diagnosis established in step c). Such a recommendation has been set forth elsewhere herein in detail.

In an aspect of the aforementioned method, steps b) and/or c) are carried out by an evaluation unit as set forth elsewhere herein.

### Method for monitoring a subject suffering from systemic infection

The definitions given herein above, apply *mutatis mutandis* to the following (except if stated otherwise):
The present invention also relates to a method for monitoring a subject who suffers from systemic infection and from cardiac damage associated with said systemic infection, comprising the steps of:
   a) determining the amount(s) of the marker cardiac troponin and/or of the marker H-FABP (Heart-type fatty acid binding protein) in a first sample and in second sample from said subject, wherein said second sample has been obtained after said first sample, and
   b) comparing the amount(s) of the marker(s) in said first sample to the amount(s) of said marker(s) in said second sample.

Preferably, an increase of the amount(s) of said marker(s) in the second sample as compared to the amount(s) of said marker(s) in said first sample indicates progression of cardiac damage. Preferably, a decrease of the amount(s) of said marker(s) in the second sample as compared to the amount(s) of said marker(s) in said first sample indicates an improvement of the condition of the subject with respect to cardiac damage. If no change is observed, i.e. an essentially identical amounts are determined in the first and the second sample, the condition of the subject is, preferably, is unchanged.

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "monitoring" as referred to above relates to keeping track in a subject of changes of cardiac damage associated with systemic infection. The term "cardiac damage" has been described elsewhere herein. The condition of the subject with respect to cardiac damage may become worse and, thus, there may be progression of damage, if the amount(s) of the biomarker(s) increase(s) whereas there is amelioration and, thus, improvement of the condition if the amount(s) ofbiomarker(s) decrease(s). If no change is observed, i.e. an essentially identical amount is determined in the first and the second sample, the condition is unchanged. An essentially identical amount is determined if no statistically significant change in the amount is determined between the first and the second sample. Whether the amounts are essentially identical can be determined by the skilled artisan without further ado.

In the context of the aforementioned method, the second sample shall have been obtained after the first sample. It is particularly contemplated that the second sample is obtained after a reasonable period of time after obtaining the first sample. It is to be understood, that the amounts of biomarkers referred herein, do not instantly change (e.g. within 1 minute or 1 hour). Therefore, "reasonable" in this context refers to intervals between obtaining the first and second sample which intervals allow the biomarker(s) to adjust. Therefore, the second sample is, preferably, obtained within three hours (in particular after three hours), more preferably, within six hours (in particular after six hours), even more preferably, twelve hours (in particular after twelve hours), or more preferably within 24 hours after the first sample (in particular after 24 hours). It is also envisaged that the second sample has been obtained after two or three days after the first sample.

It is also envisaged to assess the time course of the amount of a cardiac Troponin and/or of H-FABP in a subject suffering from systemic infection and from cardiac damage associated with systemic infection. Accordingly, the aforementioned method may comprise the additional step of determining the amount of a cardiac Troponin and/or H-FABP in at least one further sample from said subject (thus, in a third sample, in a fourth sample, in a fifth sample etc.) and comparing the, thus, determined amount with the amount of said cardiac Troponin and H-FABP, respectively, in said first sample and/or said second sample and/or any sample that was obtained before said at least one further sample was obtained. For preferred time intervals for obtaining the samples, please see above.

Preferably, an increase and, more preferably, a significant increase, and, most preferably, a statistically significant increase of the amount of a cardiac Troponin and/or H-FABP in the second sample compared with the first sample indicates progression of cardiac damage, i.e. that the condition of the subject with respect to the cardiac damage became worse. Preferably, a decrease and, more preferably, a significant decrease, and, most preferably, a statistically significant decrease of the amount of a cardiac Troponin and/or H-FABP in the second sample compared with the first sample indicates improvement of the condition of the subject with respect to cardiac damage, in particular with respect to cardiac damage associated with systemic infection.

Particularly, a significant increase is an increase of a size which is considered to be significant for the assessment, particularly said increase is considered statistically significant.

The terms "significant" and "statistically significant" are known by the person skilled in the art. Thus, whether an increase or decrease is significant or statistically significant can be determined without further ado by the person skilled in the art using various well known statistic evaluation tools.

Preferred significant increases and/or decreases of the amount of a cardiac Troponin and/or of H-FABP, preferably of the amount of the biomarker(s) in a blood, serum or plasma sample, which have been found in the course of the invention to be indicative for progression or amelioration of cardiac damage are given herein below.

According to the invention, an increase of the amount of a cardiac Troponin in the second sample compared to the amount in the first sample (or in a sample compared with the amount in a sample that was obtained earlier), preferably, of at least 3 pg/ml, more preferably of at least 4 pg/ml and even, more preferably, of at least 5 pg/ml, of at least 7 pg/ml, or of at least 8 pg/ml, of at least 12 pg/ml and most preferably of at least 15 pg/ml is considered to be significant and, thus, to indicate progression of cardiac damage, in particular of cardiac necrosis.

If the percentage increase is determined, an increase of the amount of a cardiac Troponin in the second sample compared to the amount in the first sample, preferably, of at least 15 %, of at least 20 %, more preferably of at least 40 %, and even more preferably, of at least 60 %, of at least 80 %, of at least 100 % and most preferably of at least 200 % is considered to be significant and, thus, to indicate progression of cardiac damage, in particular of cardiac necrosis.

According to the invention, an increase of the amount of a H-FABP in the second sample compared to the amount in the first sample (or in a sample compared with the amount in a sample that was obtained earlier), preferably, of at least 500 pg/ml, more preferably of at least 750 pg/ml and even, more preferably, of at least 1000 pg/ml, and most preferably of at least 1500 pg/ml is considered to be significant and, thus, to indicate progression of cardiac damage, in particular of cardiac dysfunction.

If the percentage increase is determined, an increase of the amount of a H-FABP in the second sample compared to the amount in the first sample, preferably, of at least 15 %, of at least 20 %, more preferably of at least 40 %, and even more preferably, of at least 60 %, of at least 80 %, of at least 100 % and most preferably of at least 200 % is considered to be significant and, thus, to indicate progression of cardiac damage, in particular of cardiac dysfunction.

According to the invention, a decrease of the amount of a cardiac Troponin in the second sample compared to the amount in the first sample (or in a sample compared with the amount in a sample that was obtained earlier), preferably, of at least 3 pg/ml, more preferably of at least 4 pg/ml and even, more preferably, of at least 5 pg/ml, of at least 7 pg/ml, or of at least 8 pg/ml, of at least 12 pg/ml and most preferably of at least 15 pg/ml is considered to be significant and, thus, to indicate amelioration of cardiac damage, i.e. an improvement of the condition of the subject with respect to cardiac damage, in particular with respect to cardiac necrosis

If the percentage decrease is determined, a decrease of the amount of a cardiac Troponin in the second sample compared to the amount in the first sample, preferably, of at least 5 %, of at least 10 %, more preferably of at least 15 %, and even more preferably, of at least 20 %, of and most preferably of at least 25 % is considered to be significant and, thus, to indicate amelioration of cardiac damage, i.e. an improvement of the condition of the subject with respect to cardiac damage, in particular with respect to cardiac necrosis.

According to the invention, a decrease of the amount of a H-FABP in the second sample compared to the amount in the first sample (or in a sample compared with the amount in a sample that was obtained earlier), preferably, of at least 500 pg/ml, more preferably of at least 750 pg/ml and even more preferably, of at least 1000 pg/ml, and most preferably of at least 1500 pg/ml is considered to be significant and, thus, to indicate amelioration of cardiac damage, i.e. an improvement of the condition of the subject with respect to cardiac damage, in particular with respect to myocardial dysfunction.

If the percentage decrease is determined, a decrease of the amount of a H-FABP in the second sample compared to the amount in the first sample, preferably, of at least 15 %, of at least 20 %, more preferably of at least 40 %, and even more preferably, of at least 60 %, of at least 80 %, of at least 100 % and most preferably of at least 200 % is considered to be significant and, thus, to indicate amelioration of cardiac damage, i.e. an improvement of the condition of the subject with respect to cardiac damage, in particular with respect to myocardial dysfunction.

The definitions given herein above, apply *mutatis mutandis* to the following (except if stated otherwise):
The present invention also relates to a method for assessing in a subject suffering systemic infection and cardiac damage, whether the cardiac damage is predominantly cardiac dysfunction or predominantly cardiac necrosis, the method comprising the steps of:
   a) determining the amounts of the marker cardiac troponin and the marker H-FABP (Heart-type fatty acid binding protein) in a sample from the subject
   b) calculating a ratio of the amounts of the cardiac troponin and H-FABP, and
   c) assessing whether the cardiac damage is predominantly cardiac dysfunction or predominantly cardiac necrosis.

The ratio calculated in step b) can be the ratio of amount of the marker cardiac troponin to the amount of the marker H-FABP, or the ratio of the amount of the marker H-FABP to the amount of the marker cardiac troponin.

Preferably, the ratio calculated in step b) is compared in a further step b1) to a reference ratio of the amount of the cardiac troponin and H-FABP. Preferably, if the ratio is the ratio amount of the marker cardiac troponin to the amount of the marker H-FABP, the reference ratio is also a ratio of an amount of the marker cardiac troponin to the amount of the marker H-FABP. Preferably, if the ratio is the ratio amount of the marker H-FABP to the amount of the marker cardiac troponin, the reference ratio is also a ratio of an amount of the marker H-FABP to the amount of the marker cardiac troponin.

If the ratio is the ratio amount of the marker cardiac troponin to the amount of the marker H-FABP, preferably, the following applies.

Preferably, the reference ratio is the ratio of the amount of the marker cardiac troponin to the amount of the marker H-FABP in a sample from a subject (or in samples from a group of subjects) known to suffer from cardiac damage which is predominantly cardiac necrosis, wherein a ratio in the sample of the test subject which is essentially the same, or which is increased as compared to the reference ratio indicates that the cardiac damage in the subject is predominantly cardiac necrosis.

Also preferably, the reference ratio is the ratio of the amount of the marker cardiac troponin to the amount of the marker H-FABP in a sample from a subject (or in samples from a group of subj ects) known to suffer from cardiac damage which is predominantly cardiac dysfunction, wherein a ratio in the sample of the test subject which is essentially the same, or which is decreased as compared to the reference ratio indicates that the cardiac damage in the subject is predominantly cardiac dysfunction.

The present invention also relates to the use of H-FABP and/or a cardiac Troponin or detection agents which specifically bind thereto in a sample of a subject suffering from systemic infection for diagnosing whether the subject suffers cardiac damage associated with systemic infection. Moreover, also encompassed is the use of H-FABP and/or a cardiac Troponin or detection agents which specifically bind thereto in a sample of a subject suffering from systemic infection for the manufacture of a pharmaceutical or diagnostic composition for diagnosing whether the subject suffers from cardiac damage associated with systemic infection or not.

The present invention also relates to the use of H-FABP and/or a cardiac Troponin or detection agents which specifically bind thereto in a first and second sample of a subj ect suffering for monitoring cardiac damage in a subject suffering from systemic infection. Moreover, also encompassed is the use of H-FABP and/or a cardiac Troponin or detection agents which specifically bind thereto in a first and second sample of a subject suffering from systemic infection for the manufacture of a pharmaceutical or diagnostic composition for monitoring cardiac damage.

The term "detection agent" as used herein refers to an agent that is capable of specifically recognizing and binding to the biomarker polypeptide(s) present in a sample. Moreover, the said agent shall allow for direct or indirect detection of the complex formed by the said agent and the biomarker. Direct detection can be achieved by including into the agent a detectable label. Indirect labelling may be achieved by a further agent that specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

The present invention also relates to a device adapted for carrying out a method of the invention comprising
a) an analyzing unit comprising a detection agent which specifically binds to H-FABP and/or of a detection agent which specifically binds to a cardiac troponin , said unit being adapted for determining the amount of H-FABP and/or of the cardiac troponin in a sample of a subject suffering from systemic infection; and
b) an evaluation unit for comparing the determined amount(s) with reference amount(s) whereby it can be diagnosed whether the said subject suffers from cardiac damage associated with systemic infection, said unit comprising a database with reference amount values and a computer-implemented algorithm carrying out the comparison.

Preferred reference amounts and algorithms are disclosed elsewhere herein.

The term "device" as used herein relates to a system comprising the aforementioned units operatively linked to each other as to allow the diagnosis or monitoring according to the methods of the invention. Preferred detection agents which can be used for the analysing unit are disclosed elsewhere herein. The analysing unit, preferably, comprises said detection agents in immobilized form on a solid support which is to be contacted to the sample comprising the biomarkers the amount of which is to be determined. Moreover, the analysing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references are either derived from a subject or group of subjects as defined above in context with the method of the present invention. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician.

It follows from the above that according to some embodiments of the instant disclosure, portions of some steps of methods disclosed and described herein may be performed by a computing device. A computing device may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), such as BLACKBERRY brand devices, cellular devices, tablet computers, servers, and the like. In general, a computing device comprises a processor capable of executing a plurality of instructions (such as a program of software).

A computing device has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and non-volatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device. According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art. The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The computing device may also have access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Finally, the invention pertains to a kit adapted for carrying out a method of the present invention comprising a detection agent for H-FABP and/or a detection agent for a cardiac troponin, reference standards as well as instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Further, the kit shall comprise at least one standard for a reference as defined herein above, i.e. a solution with a pre-defined amount for the H-FABP representing a reference amount and/or for a cardiac troponin representing a reference amount. Such a standard may represent, e.g., the amount of the subject from a subject or group of subjects suffering from systemic infection and suffering from cardiac damage associated with said systemic infection or a subject or group of subjects suffering from systemic infection and not suffering from cardiac damage associated with systemic infection.

In some embodiments, a kit disclosed herein includes at least one component or a packaged combination of components for practicing a disclosed method. By "packaged combination" it is meant that the kits provide a single package that contains a combination of one or more components, such as probes (for example, an antibody), controls, buffers, reagents (for example, conjugate and/or substrate) instructions, and the like, as disclosed herein. A kit containing a single container is also included within the definition of "packaged combination." In some embodiments, the kits include at least one probe, for example an antibody (having specific affinity for an epitope of a biomarker as disclosed herein. For example, the kits may include an antibody that is labelled with a fluorophore or an antibody that is a member of a fusion protein. In the kit, the probe may be immobilized, and may be immobilised in a specific conformation. For example, an immobilized probe may be provided in a kit to specifically bind target protein, to detect target protein in a sample, and/or to remove target protein from a sample.

According to some embodiments, kits include at least one probe, which may be immobilized, in at least one container. Kits may also include multiple probes, optionally immobilized, in one or more containers. For example, the multiple probes may be present in a single container or in separate containers, for example, wherein each container contains a single probe.

In some embodiments, a kit may include one or more non-immobilized probe and one or more solid support that does or does not include an immobilized probe. Some such embodiments may comprise some or all of the reagents and supplies needed for immobilizing one or more probes to the solid support, or some or all of the reagents and supplies needed for binding of immobilized probes to specific proteins within a sample.

In certain embodiments, a single probe (including multiple copies of the same probe) may be immobilized on a single solid support and provided in a single container. In other embodiments, two or more probes, each specific for a different target protein or a different form of a single target protein (such as a specific epitope), a provided in a single container. In some such embodiments, an immobilized probe may be provided in multiple different containers (e.g., in single-use form), or multiple immobilized probes may be provided in multiple different containers. In further embodiments, the probes may be immobilized on multiple different type of solid supports. Any combination of immobilized probe(s) and container(s) is contemplated for the kits disclosed herein, and any combination thereof may be selected to achieve a suitable kit for a desired use.

A container of the kits may be any container that is suitable for packaging and/or containing one or more components disclosed herein, including for example probes (for example, an antibody), controls, buffers, and reagents (for example, conjugate and/or substrate). Suitable materials include, but are not limited to, glass, plastic, cardboard or other paper product, wood, metal, and any alloy thereof. In some embodiments, the container may completely encase an immobilized probe(s) or may simply cover the probe to minimize contamination by dust, oils, etc., and expose to light. In some further embodiments, the kits may comprise a single container or multiple containers, and where multiple containers are present, each container may be the same as all other containers, different than others, or different than some but not all other containers.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1: Patient cohorts

A total of 172 patients presenting with signs of SIRS and suspected infection were included into the study, they were graded according to the APACHE II score and outcome at 30 days was recorded.

As reference population, 149 apparently healthy persons, who has no evidence of cardiac disease based on physical examination. medical history and laboratory tests, moreover they has no evidence of chronic or acute infection, in addition they had normal kidney function.

A second reference population consisted of 90 patients with stable coronary artery disease and classified as NYHA I or II according to the NYHA classification. Coronary artery disease was confirmed by angiography, kidney function was within normal.

A third control group consisted of 117 patients with decompensated heart failure from chronic heart failure, patients has no obvious coinfection, creatinine values were within normal.

### Example 2: Determination of Troponin T, NT-proBNP and of H-FABP

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac Troponin T. The antibodies recognize two epitopes (amino acid position 125-131 and 136-147) located in the central part of the cardiac Troponin T protein, which consists of 288 amino acids (analytical sensitivity below 1,0).

NT-proBNP was determined using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

H-FABP was tested by using the H-FABP ELISA Kit for human heart type fatty acid binding protein (catalogue no.: HK402 or HK401), of HyCult Biotechnology, Uden, The Netherlands. This test allows for quantitative determination of H-FABP in a sample.

### Example 3: Results

### apparently healthy controls

The results are shown in the following table

| | NT-pro BNP | H_FABP | Troponin T |
|---|---|---|---|
| | pg/ml | pg/ml | pg/ml |
| N = 149 | 37(18/67) | 1483 (1257/1793) | 0 (0/0) |
| indicated are the medians as well as the 25. and 75. percentiles (0 = below 1 pg/ml) | | | |

### Patient with stable coronary artery disease

The results are shown in the following table

| | NT-pro BNP | H-FABP | Troponin T |
|---|---|---|---|
| | pg/ml | pg/ml | pg/ml |
| Total group | | | |
| N=90 | 129 (57/238) | 2207 (1688/2968) | 1,3 (0,0/8,1) |
| Below Median | 54 (35/95) | 2033 (1599/2412) | 1,0 (0,0/6,0) |
| N=45 | | | |
| Above Median | 241 (175/313) | 2427 (1706/3334) | 3(0/10) |

### Patients with decompensated heart failure

The results are shown in the following table

| | NT-pro BNP | H-FABP | Troponin T |
|---|---|---|---|
| | Pg/ml | pg/ml | pg/ml |
| Total group | 4478 (2187/7413) | 6427(4234/9608) | 23 (14/46( |
| N = 117) | | | |
| 1. Tertile | 1350 (907/2161) | 6327(4176/8005) | 18 (10/25) |
| N=39 | | | |
| 2. Tertile | 4478 (3676/5250) | 4975 (3697/8757) | 23 (16/39) |
| N=39 | | | |
| 3. Tertile | 9827 (7660/13267) | 7615 (5102/10464) | 42 (21/54) |

As can be seen from the Table patients with different severity of cardiac disease and specifically heart failure has lower H-FABP values at similar NT-proBNP levels than patients with proven or suspected systemic infection indicating that H-FABP concentrations can be used to guide to diagnose "inflammatory heart disease" (even in patients with pre-existing heart failure).

### SIRS population

NT-pro BNP, Troponin T and H-FABP were analyzed according to the Quartiles of APACHE II Score, the results are as follows:

| APACHE II | NT-pro BNP | H-FABP | TroponinT | died |
|---|---|---|---|---|
| Score | Pg/ml | pg/ml | pg/ml | n |
| 7 | 146 (55 - 265) | 2378 (1700 - 4587) | 5 (3 - 15) | 0 |
| 13 | 841 (256/1372) | 6213 (3549 - 9052) | 12 (9 - 27) | 1 |
| 17 | 1678 (551 - 4599) | 10591 (7177 - 13977) | 25 (14 - 53) | 3 |
| 26 | 7793 (2342 - 21963) | 27774 (16235 - 68086) | 80 (40 - 160) | 6 |

As can be seen markers of cardiac function, markers of myocardial dysfunction and myocardial necrosis increased with increasing APACHE II Scores.

Individual correlation of NT-pro BNP, Troponin T and H-FABP to the APACHE II Score indicates that there is no stringent correlation. This indicates that markers for cardiac function (NT-pro BNP), cardioac necrosis (Troponin T) und cardaic dysfunction (H-FABP) provide independent information that can be used for clinical assessment and treatment stratification.

The correlation of Troponin T to H-FABP indicates that both markers correlate. The correlation is however not stringent. Rather, the correlation indicates that in individual patients H-FABP (cardiac dysfunction) whereas in others necrosis (troponin T) predominates.

In an additional analysis survivors and non-survivors at 30 days were assessed separately, this is shown in the Table below:

| | Survivor | Non Survivor |
|---|---|---|
| | N = 161 | N= 11 |
| NT-pro BNP pg/ml | 949 (218 - 3650) | 20712 (6552 - 37428) |
| Troponin T | 18 (8 - 51) | 83 (27 - 202) |
| H-FABP | 8021 (1918 - 17441) | 20713 (14161 - 37739) |
| H-FABP/Trop T | 398 (260 - 670) | 298 (221 - 739) |

As can be seen all markers were significantly higher in non survivors than in survivors except the H-FABP/Troponin T ratio indicating that in survivors cardiac dysfunction was more predominant and thus reversible and in non survivors who showed relatively more necrosis which is irreversible.

Follow up of systemic infection, patients suffering from pneumonia and systemic infection were also included into the study, samples were taken at 24 h interval.

| Time | NT-pro BNP | HFABP | Troponin T | PCT |
|---|---|---|---|---|
| | Pg/ml | pg/ml | pg/ml | ng/ml |
| Case 11 0 | 5067 | 19576 | 57 | 2,49 |
| Case 11 24 h | 2602 | 10079 | 43 | 1,74 |
| Case 11 48 h | 2245 | 5013 | 25 | 0,89 |
| Case 28 0 | 6364 | 9401 | 17 | 0,02 |
| 28 24 h | 17896 | 25531 | 100 | 0,51 |
| 28 48 h | 26357 | 92165 | 96 | 0,6 |
| Case 53 0 | 2955 | 23348 | 112 | 1,5 |
| 53 24 h | 5458 | 19449 | 138 | 3,8 |
| 53 48 h | 4197 | 8545 | 150 | 3,0 |
| Case 09 0 h | 6352 | 2476 | 78 | 0,02 |
| 24 h | 6474 | 8352 | 106 | 0,02 |
| 48 h | 5392 | 9089 | 93 | 0,02 |
| 72 h | 7346 | 8785 | 120 | 0,02 |
| Case 159 0 h | 4580 | 12177 | 65 | 0,3 |
| 159 24 h | 14566 | 13876 | 167 | 0,6 |
| 159 48 h | 30289 | 62924 | 517 | 1,2 |
| Case 51 0 | 862 | 1738 | 6 | 0,056 |
| 51 24 h | 1595 | 2436 | 10 | 0,056 |
| 51 48 h | 1535 | 1881 | 9 | 0,068 |
| 51 72 h | 1168 | 1712 | 8 | 0,049 |

The examples illustrate 6 cases of pneumonia with systemic infection that were followed at 24 h interval. Procalcitonin was used to confirm or rule out bacterial infection. All but two cases (09 /51) had evidence of bacterial infection based on PCT values. Case 09 has pneumonia and underlying heart failure (on medical history) ,this is also reflected by relatively low H-FABP levels. This was also the case of example 51, this patients had less severe heart failure when compared to patients 09. All other patients had bacterial pneumonia with different levels of PCT and different dynamics of H-FABP und Troponin T. The fact that H-FABP and troponin T can provide supplemental information can be obtained from case 53, where H-FABP followed the decrease of NT-pro BNP. In most cases the dynamics of H-FABP were greater than that of Troponin T indicating the significant (discriminatory) clinical value of H-FABP in follow up.

### Example 4: Cases

42 year old male, who was previously healthy develops fever after a recent upper respiratory infection and dizziness, he went to the emergency room and chest x ray revealed pneumonia, his NT-pro BNP was 2680 pg /ml, FABP was 1400 pg7ml and Troponin T was 32 pg/ml. He was diagnosed with systemic infection and an antibiotic therapy was started, fluid rescutation was not needed at this time, the patient recoverd and after 7 days NT-pro BNP was 110 pg/ml, H FABP was 1850 pg/ml and Troponin T was not detectable by the sensitive troponin T test.

A 36 year old female develops fever and goes to her physician because of headache, her NT-pro BNP is 64 pg/ml, H-FABP is 1560 pg/ml and troponin T was undetectable. based on the above laboratory tests a viral infection was assumed and a systemic infection excluded. The patient recovered without antibiotic therapy.

A 64 year old patient with known heart failure develops dizziness und insomnia, he has a history of heart failure and took beta blockers, ACE inhibitors and diuretics. Because of the above symptoms he goes to the emergency room on a weekend, his NT-pro BNP is 4500 pg/ml, H-FABP is 13000 pg/ml and Troponin T is 52 pg/ml. He is diagnosed with superimposed systemic infection, treated with antibiotics and carefully receives fluids. On Monday, his home physician is called. He tested the patient for NT-pro BNP and H FABP as well as troponin T 2 weeks ago: NTproBNP was 865, H-FABP was 4230 and Troponin T was 13 pg/ml, further strengthening the suspected diagnosis of cardiac disease due to systemic infection.

A 68 year old patient with heart failure develops fever and hypotension and goes to the emergency room. His NT-proBNP is 1280, Troponin T is 8 pg/ml and H-FABP is 3980 pg/ml. He is diagnosed with viral infection in chronic heart failure and his heart failure therapy is adopted.

### Conclusion:

The determination of H-FABP in addition to Troponin T offers important information related to cardiac damage in systemic infection where H-FABP represent a marker of cardiac dysfunction which is reversible and Troponin T as marker of cardiac necrosis which is irreversible. H-FABP and to a lesser extent Troponin T are higher in cardiac disease caused by infection than in heart failure patients without infection (when related to NT-pro BNP values). This has impact on treatment (e.g. the use of fluid rescutation and antibiotics in heart disease associated with systemic infection in addition to heart failure therapy (in indicated) in patients without systemic infection. This information can also be used to monitor patients with systemic infection and to adapt treatment strategies.

In summary the present invention is useful in the characterization of patients with cardiac disease and evidence of infection and in making treatment decision.

## Claims

1. A method for diagnosing in a subject suffering from systemic infection whether cardiac damage is associated with systemic infection, comprising the steps of
a) determining the amount(s) of the marker cardiac troponin and/or of the marker H-FABP (Heart-type fatty acid binding protein) in a sample from said subject, and
b) comparing the, thus, determined amount(s) to a reference amount (reference amounts), thereby diagnosing whether cardiac damage is associated with systemic infection,
wherein (i) the reference amount(s) is (are) derived from a reference subject suffering from systemic infection and pre-existing heart disease, if the subject to be tested suffers from pre-existing heart disease, and/or wherein (ii) the reference amount(s) is (are) derived from a reference subject suffering from systemic infection, but not suffering from pre-existing heart disease, if the subject to be tested does not suffer from pre-existing heart disease.

2. The method of claim 1, wherein the cardiac damage is cardiac necrosis and/or cardiac dysfunction.

3. The method of claims 1 and 2, wherein the pre-existing heart disease is coronary heart disease and/or heart failure.

4. The method of any one of claims 1 to 3, wherein the reference subject is known not to suffer from cardiac damage associated with systemic infection, and wherein an amount of the marker(s) in the sample from the subject to the tested which is (are) essentially identical or which is (are) decreased as compared to the reference amount(s) indicates that the subject to be tested does not suffer from cardiac damage associated with systemic infection.

5. The method of any one of claims 1 to 4, wherein the reference subject is known to suffer from cardiac damage associated with systemic infection, and wherein an amount of the marker(s) in the sample from the subject to the tested which is (are) essentially identical or which is (are) increased as compared to the reference amount(s) indicates that the subject to be tested suffers from cardiac damage associated with systemic infection.

6. The method of claim 5, wherein the marker is a cardiac troponin, and wherein the cardiac damage is cardiac necrosis.

7. The method of claim 6, wherein the marker is H-FABP, and wherein the cardiac damage is cardiac dysfunction.

8. The method of any one of claims 1 to 7, further comprising the step of recommending a therapy, if the subject to be tested suffers from cardiac disease associated with systemic infection.

9. The method of any one of claims 1 to 8, wherein both the amounts of the markers cardiac troponin and H-FABP are determined in the sample from the subject.

10. A method for monitoring a subject who suffers from systemic infection and from cardiac damage associated with said systemic infection, comprising the steps of:
a) determining the amount(s) of the marker cardiac troponin and/or of the marker H-FABP (Heart-type fatty acid binding protein) in a first sample and in second sample from said subject, wherein said second sample has been obtained after said first sample, and
b) comparing the amount(s) of the marker(s) in said first sample to the amount(s) of said marker(s) in said second sample.

11. The method of claim 10, wherein an increase of the amount(s) of said marker(s) in the first sample as compared to the amount(s) of said marker(s) in said second sample indicates progression of cardiac damage, and/or wherein a decrease indicates an improvement of the condition of the subject with respect to cardiac damage.

12. The method of any one of claims 1 to 11, wherein the sample is blood, serum or plasma.

13. The method of any one of claims 1 to 12, wherein the subject is human.

14. Use of H-FABP and/or a cardiac Troponin or detection agents which specifically bind thereto in a sample of a subject suffering from systemic infection for diagnosing whether the subject suffers cardiac damage associated with systemic infection, or use of H-FABP and/or a cardiac Troponin or detection agents which specifically bind thereto in a first and second sample of a subj ect suffering from systemic infection for monitoring cardiac damage in said subject.

15. A device adapted for carrying out a method of any one of claims 1 to 9 comprising
a) an analyzing unit comprising a detection agent which specifically binds to H-FABP and/or of a detection agent which specifically binds to a cardiac troponin , said unit being adapted for determining the amount of H-FABP and/or of the cardiac troponin in a sample of a subject suffering from systemic infection; and
b) an evaluation unit for comparing the determined amount(s) with reference amount(s) whereby it can be diagnosed whether the said subject suffers from cardiac damage associated with systemic infection, said unit comprising a database with reference amount values and a computer-implemented algorithm carrying out the comparison.
